# EUROPEAN PATENT APPLICATION

(11) **EP 3 786 119 A1**
(43) Date of publication of application: **03.03.2021**
(21) Application number: 19460045.8
(22) Date of filing: 31.08.2019
(51) Int. Cl.: C02F 3/28, C02F 11/04, C12M 1/00, C12P 5/02, B01D 61/00, B01D 65/02, C12M 1/34, B01D 63/06

(54) **METHOD OF OBTAINING BIOGAS IN ANAEROBIC BIOLOGICAL WASTEWATER TREATMENT PLANT, AND A REACTOR FOR OBTAINING BIOGAS AND WASTEWATER TREATMENT**

(71) Applicant: IMA Polska, 62-095 Murowana Goslina (PL)
(72) Inventor: IMA, Krzysztof, 64-400 Oborniki (PL)

(57) **Abstract**

The invention concerns a method of biogas production in an anaerobic biological wastewater treatment plant, and a reactor (1) for biogas production and wastewater treatment. The invention can be applied as a compact, medium-loaded treatment system, which combines biological degradation with a separation process, wherein the separation concerns fractions of heavy substrates taking part in the reaction. In particular, the substrate that is dosed in the reaction is the stillage leachate remaining after decantation. It is preferable for the invention to have a further possibility to generate energy from the biogas, mostly from methane, which is a main component of biogas produced with the invention, and the possibility to significantly utilize the harmful substances in the process according to the invention.

The reactor (1) for biogas production and wastewater treatment includes two tanks (3) interconnected with transmission pipelines (2), the first of which is the buffer tank (3') and the second is the main tank (3"), where both tanks are provided with a temperature sensor (4), pH sensor (5) and pressure sensor (6). The tanks (3) are equipped with mixing modules (7) that include a rotor (8). The biogas outlet (23) is located at the top of each tank (3', 3"). The flow of agents through the tanks (3) and transmission pipelines (2) is controlled by a control system (9), based on the readouts of sensors (4, 5, 6). The mixing modules (7) are located outside the tanks (3) and are each time connected to the tanks (3) with their own pipeline circuit system (10), which has a turbulent circulation pump (11), and the main tank (3") is additionally connected to a membrane filter system (14) through an external turbulent pipeline circuit (13) forced by the second pump (12). The membrane filter system (14) is connected by its outlet to the core (15) running centrally through the main tank (3"), generally at half height of the core (15), which core (15) is opened on top to obtain overflow into the main tank (3"), and at the bottom it is terminated with an outlet (16) for concentrated sludge. The membrane filter system (14) has its own outlet (17) for filtrate in the form of treated leachate.

## Description

The invention concerns a method of biogas production in an anaerobic biological wastewater treatment plant, and a reactor for biogas production and wastewater treatment. The invention can be applied as a compact, medium-loaded treatment system, which combines biological degradation with a separation process, wherein the separation concerns fractions of heavy substrates taking part in the reaction. In particular, the substrate that is dosed in the reaction is the stillage leachate, which is a production residue. It is preferable for the invention to have a further possibility to generate energy from the biogas, mostly from methane, which is a main component of biogas produced with the invention, and the possibility to significantly utilize the harmful substances in the process according to the invention.

From the international application No. PCT/BE2002/000168, proceeded also in Poland under number P.369461, we know a process of methanation and acetogenesis, used as the second stage in the technology for irreparable destruction of securities. However, at the first stage securities are cut and/or shredded in the process of hydrolysis and acidogenesis, for which the pH maintains below 6, at 30°C to 60°C, preferably at 30°C to 40°C. Then the securities are gradually moved to one or more methanation and acetogenesis tanks, where the pH is kept at 7.2 to 8 using basic substances or buffers, so that this environment causes fermentation of the securities surrounded by anaerobic bacteria at 50°C to 60°C. As a result, at the second stage we obtain methane and carbon dioxide, but at the first stage the part of the semi-finished product that was moved to the second stage is immediately replaced by a mixture of securities which were not pretreated and come from the shredding and mixing equipment. However, this two-stage process is not oriented to methane production, which is in a way only generally useful, but not intentional. The efficiency of so produced methane does not seem high. What is useful is the two-stage process and the replacement of partially reacted charge with the charge being delivered, which is just entering the chemical reaction.

Therefore, from the Polish application number P.420700, we know a method of intentional production of dried distillate and fodder amino acids as part of bio-ethanol production. In this case, the essence of the known solution is that the raw materials (starch and sugar biomass) are shredded and then mixed with water and stabilised, and next the starch contained in the raw materials undergoes enzymatic hydrolyse in order to obtain fermenting sugars, but apart from the starch the degradation also covers non-starch ingredients of the raw material, which, combined with water, create high-viscosity gels. Next, the mixture is complemented with sodium hydroxide, and then we add an enzymatic preparation with active xylanase, which causes propagation in a mash environment with a high FAN concentration, and then we dose sulphur acid, and then we add an enzymatic preparation with active amyloglucosidase with secondary activities of alpha-amylase and phytase, and we carry out the fermentation process. Then we proceed with distillation at a concentration of ethanol in the liquid prior to the distillation, wherein the produced ethanol is emitted by the post-process mixture by way of distillation, and the proteins contained in the stillage are degraded to the form of peptides and amino acids using enzymatic hydrolysis. Then, according to the known process, we return the hydrolysate to the fermentation process in order to make the alcohol fermentation and propagation more dynamic by returning the hydrolysate in the form of a dry matter of the mixture prior to separation, but the emitted suspended matter constitutes a dried distillate containing protein hydrolysate, and the liquid fraction is carried to the stage of isolation of amino acids, which are separated from the solution, either as a commercial mixture by membrane filtration and crystallisation to a selected salt, or they are separated into single amino acids using first the membrane filtration for agent purification and then separation with the use of industrial ion-exchange chromatography, and finally they are crystallised to a selected salt. Then, according to the known process, the emitted amino acid fraction is dried to obtain a solid product in the form of amino acid mixture or a selected preparation of a specific amino acid, and then all leachates produced throughout the process are subjected to anaerobic treatment and then to aerobic treatment with the activated sludge method, and the biogas so produced is used for energy generation. As a rule, the process described actually shows that, at the final stage, we can produce biogas, which may be used to generate energy, but the issue with that technology still consists in a high concentration of contaminants and chemical components, in particular the so-called COD and BOD, as well as a low efficiency of the production of biogas or the mere methane contained in the biogas.

From the Polish application number P.393015, we know a method of producing biogas from sewage sludge by methane fermentation, wherein the line supplying the sewage sludge to the anaerobic reactor is fed with 10%-20% (preferably 10%) by volume of distilling waste and with 80%-90% (preferably 90%) of sewage sludge. Due to the combination of two types of substrate, which may be a source of methane, the process is faster than in other known methods of methane production, but even so the anaerobic reaction in the anaerobic reactor needs as many as 16 to 18 days to produce 0.8m³ of biogas per 1kg of extracted organic suspended matter and 0.5m³ of biogas per 1kg of extracted COD compound, and the content of methane in the biogas is only 55%. As we can see, the known method, even if reasonable, is neither very fast given the fermentation time nor sufficiently efficient given the amount of biogas produced by the fermentation.

From the Polish patent number PAT.213348, we know a method of generating electrical and thermal energy, fertilisers and ethanol from crops and production waste generated in the agricultural industry using waste, in particular such as stillage, animal excrements, straw, rape cake, ensilage and other substrates. The known method is based on the integration of: a distillery with a biogas plant, heat take-off points at the distillery and the conversion of stillage into energy, fertiliser and/or animal feed, with the aim to obtain synergy in the processing of production waste, both in terms of economy and ecology. The method uses crops and production waste to make such products as ethanol, electrical and thermal energy, fertilisers or fodders. It may be widely used in the agrifood industry. The known method allows for biogas production mostly from stillage, with possible additives, but it needs a multi-staged process, which is complicated and consumes time and energy.

The solution according to this invention is based on known prior methods but, surprisingly, it allows for a very fast production of biogas at large quantities which considerably exceed the previously achievable efficiencies, and it allows for a very high treatment of the base material which, as a substrate, is used for the reaction to produce methane, so finally the process generally does not generate any harmful substances, and even if it does, they are returned to the process or reused for preliminary production necessary to obtain a substrate in the form of stillage leachate.

In general, the method of biogas production in an anaerobic biological wastewater treatment plant, and the reactor for biogas production and wastewater treatment, according to the invention, is based on an anaerobic biological medium-loaded reactor which uses the arrangement of ceramic membranes. The arrangement ensures treatment of medium-loaded mono-substrate having the form of stillage leachate. As the substrate is difficult to be treated due to diverse substances it contains, the structurally assembled arrangement is a compact treating system which combines biological degradation with membrane separation.

In particular, the substrate flux is directed to the anaerobic reactor, where organic compounds are subjected to methane fermentation. The process is accompanied by biogas production. During anaerobic fermentation, the fermenting substrate each time recirculates, thanks to pumps, in each module through an external mixer independent for each reactor, which also acts as a temperature stabiliser. Therefore, the essence of the solution consists in a two-staged structure of the process, which each time includes an anaerobic reaction of the substrate (stillage leachate) in two reactors (buffer and main tanks) connected in cascade, wherein in the first tank the substrate is intensively mixed, and in the second tank, besides intensive mixing, the substrate is additionally led by another pump through an external system of membrane filters independent of the mixers, and after filtration it is supplied to the core structurally separated in the second anaerobic reactor, where the supply of the filtered substrate takes place more or less at half height of the core, and the highly concentrated substrate falls down through the core and is removed from the reactor in a semi-solid state, while the medium- and low-concentrated substrate is moved up through the core and flows over to the reactor, where it then participates in the anaerobic reaction. In both reactors, the produced biogas is collected at their top outlet. The membranes are used to separate the filtrate (permeate), as a clean substance, from the biomass. As already mentioned, the biomass is returned to the core of the second reactor, while the filtrate devoid of solid components and with a low content of BOD/COD is directed for production in the existing plant or distillery, or sent to the sewage system. Suspended matters, including bacteria, and colloidal solutions are stopped at the small-pore membranes, and thus they remain in the biological reactor, but at a certain interval the membranes are preferably cleaned, which does not impair the process, as the membranes are situated outside the anaerobic reactor, and the reaction occurring in the reactor is not suspended for cleaning.

The summary of the solutions according to the invention is as follows.

The process of producing biogas in an anaerobic biological wastewater treatment plant, with the use of hydrolysis, acidogenesis, acetogenesis and methanogenesis, is carried out continuously, but in two stages, using a substrate supplied upon mixing and temperature stabilising after a prior basic production stage. The substrate is a centrifugated stillage, that is stillage leachate. At the first stage, the pH is maintained below 7, and at the second stage it is maintained above 7, and at the same time both stages are carried out at 30°C to 45°C, in tanks, the first of which is the buffer tank and the second is the main tank, and the substrate is gradually moved from the first to the second tank, while continuously making up for its loss in the first tank. The pH is maintained with basic substances or buffers, in order to use such an environment for fermentation among bacteria, so as a result we obtain biogas in the form of methane and carbon dioxide, being a product of the reaction, which emits an excess biomass. The flow of agents through tanks and transmission pipes is controlled by a control system, based on sensor readouts. The method is characterised in that the first stage, taking place in the buffer tank, includes the processes of hydrolysis, acidogenesis and acetogenesis of the leachate, and the leachate temperature during the reaction is stabilised at 38°C +/- 3°C, even though the leachate is supplied to the first tank at 30°C to 35°C (but its temperature after the basic distillation process is as high as 70°C to 80°C), and then, having equalised the flow velocity and concentration and having stabilised the temperature, we force the flow of pre-reacted substrate (ferment) to the second tank, where it is intensively linked with the retentate in a semi-liquid state, that is biomass filtered in the system of membrane filters connected to the second tank by an external forced circuit. The digestate produced in the membrane filter system is introduced to the core running centrally through the second tank, generally at half of its height, and then the excess concentrated sludge, as a digestate produced from the retentate, is removed from the reactor at the bottom of the core, and the digestate of semi-liquid phase is removed by overflow at the upper part of the core, thus feeding the second tank, and the filtrate, as a treated leachate, is removed from the membrane filter system. The leachate introduced to the tank remains therein and reacts for 24h +/- 2h on average, and then the pre-reacted substrate (ferment), supplied to the second tank, is mixed and filtered therein for the next 48h +/- 2h on average, and then it is only mixed for the next 48h +/- 2h on average, and at the same time the produced biogas is continuously collected - a full cycle of a dosed leachate sample, from its introduction to the production of biogas, takes less than 125h. It is important that the pH obtained at the first stage ranges from 5.5 to 6.5, and at the second stage - from 7 to 7.6.

Preferably, at the first or second stage (but best at the first one), nutrients are supplied, preferably N or P or FeC13.

Preferably, at the first or second stage, the pH is adjusted, best by appropriately supplying NaOH.

Preferably, the filtrate is utilized or returned to the distillery, that is to the previous basic production stage.

The biogas may be collected on a one-off basis, in cycles or in portions.

Preferably, the membrane filter system is cleaned chemically, no less than once a month, while the process is continued, and the cleaning is carried out using a secondary circuit separated from the tank system, with the forced direction being opposite (when running through the membrane filters) to the direction of flow through the membranes during a standard process.

The reactor for biogas production and wastewater treatment includes two tanks connected with transmission pipelines, the first of which is the buffer tank and the second is the main tank, where both tanks are provided with a temperature sensor, pH sensor and pressure sensor. The tanks are equipped with mixing modules that include a rotor. The biogas outlet is located at the top of each tank. The flow of agents through the tanks and transmission pipelines is controlled by a control system, based on sensor readouts. The reactor is characterised in that the mixing modules are located outside the tanks and are each time connected to the tanks with their own pipeline circuit system, which has a turbulent circulation pump, and the main tank is additionally connected to a membrane filter system through an external turbulent pipeline circuit forced by the second pump. The membrane filter system is connected by its outlet to the core running centrally through the main tank, generally at half height of the core, which core is opened on top to obtain overflow into the main tank, and at the bottom it is terminated with an outlet for concentrated sludge. The membrane filter system has its own outlet for filtrate in the form of treated leachate.

Preferably, the membrane filter system is connected to the main tank by shut-off valves.

Preferably, the membrane filter system has a second secondary circuit, used for chemical cleaning of the filters.

Preferably, the membrane filter system is provided with at least three filters in the form of membranes, located in parallel to one another, preferably in vertical position.

Preferably, the cross-section of the membrane filter system has an area of up to 1m².

Preferably, a single membrane filter is longitudinally divided inside into channels having a shape similar to a triangle or trapezoid.

Preferably, a single filter has no less than five and no more than 40 channels.

Preferably, the capacity of the main tank is no less than twofold and no more than fivefold higher than the capacity of the buffer tank but does not exceed 5,000 m³.

Preferably, any of the tanks is provided with an anti-foaming sprinkler.

Preferably, any of the tanks is provided with a sight glass, which is preferably equipped with a movable wiper.

Preferably, the main tank has at least two independent membrane filter systems.

Preferably, a membrane filter is a ceramic filter, preferably made of titanium or zirconium oxide, with a throughput of the membrane mesh of 0.01 µm to 2.0µm.

The invention meets the assumed objectives:
- biogas output is even 20% higher due to the cascade arrangement in the reactor, due to external filtration in the membrane filters, due to turbulent flows, and due to the averaging and proper preparation of medium, which features are regarded as a synergy; the absence of any of these four features does not allow to increase biogas production, which was empirically tested by removing each feature one by one or in groups; even a separation of the tanks with the other features maintained causes the produced amount of biogas to be considerably lower.
- The COD reduction gradually increases at each subsequent stage of the process, to reach even 99.5% at the last stage.

Therefore, the benefits of the solution according to the inventions mostly include:
- considerably improved quality of wastewater and leachate at the outlet due to the absence of suspended matters, and reduced concentration of colloids related to COD and BOD and to nutrients (nitrogen, phosphorus);
- considerably increased concentration of active biomass in the reactor, which allows to make the biological treatment more effective;
- increased unexpected emission of biogas, which is much higher in total than if we aggregate the emissions from two tanks operating independently and separately (buffer tank and main tank), due to a cascade design of the reactor and a good (optimum) preparation of the reacting medium transmitted to the main tank;
- additional biogas produced from the suspended fraction and biodegradable fats (ester extract), which do not need to be removed or independently filtered, as in the case of high-loaded reactors with granulated sludge;
- unique configuration of filtering membranes located outside the reactor, which provides a simple, easy to maintain, independent and closed system; this eliminates the issue with the emission of unpleasant odours; pressure in the reactor is 100-200 mbar and is maintained with a safety valve;
- considerably increased reduction of COD (95% even up to 99.5%) by what was supplied to the reactors along with the medium, which is due to no suspended matter or colloidal material existing/remaining in the filtrate flowing out of the filtration section (after passing the membranes);
- significantly smaller size of the reactor compared to currently known structures, with a several times higher load;
- an anaerobic reactor with an integrated membrane system is a process which is maintenance-free, as there is no corrosion; aside from agitators and pumps, there is no complex equipment that could disturb the operation;
- therefore, the reactor does not require any maintenance, as the membranes are installed outside it and are easily accessible for servicing;
- the system of chemical cleaning of the membranes is fully automated and does not require the membranes to be cleaned or dismantled by a specialised maintenance team. The cleaning process uses typical and inexpensive chemicals.

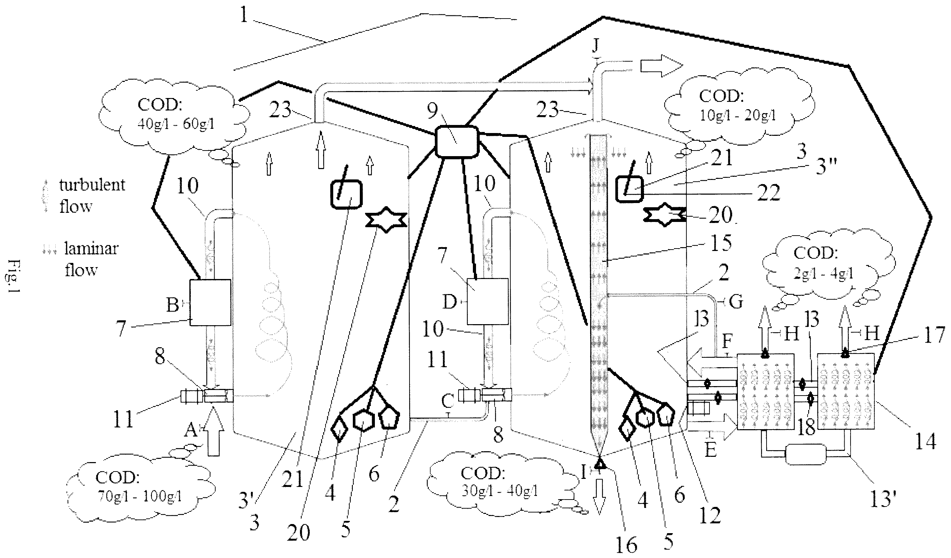

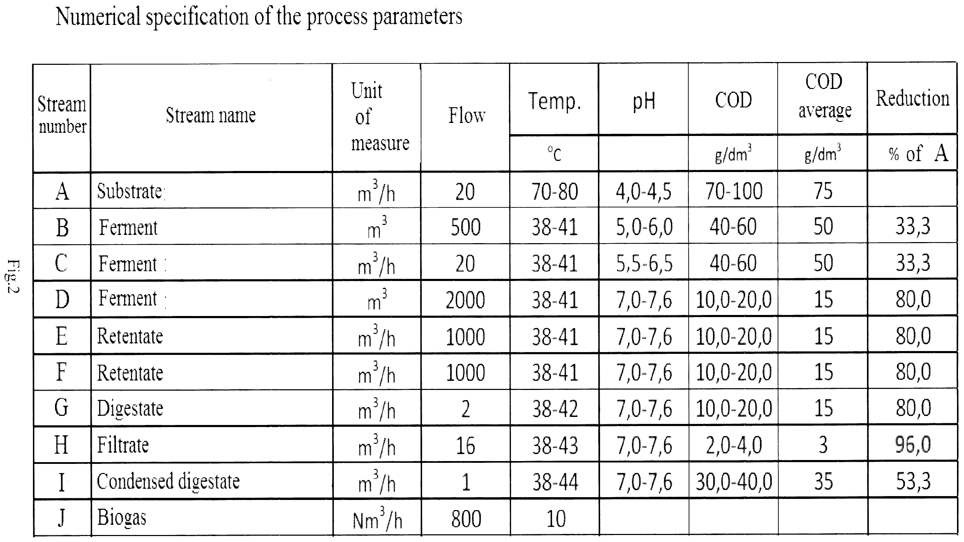

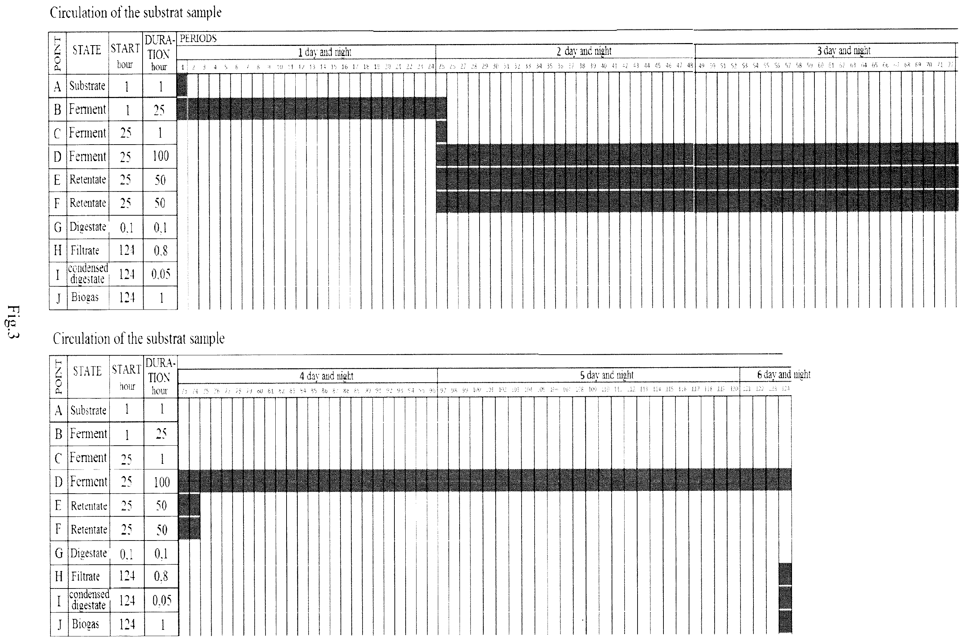

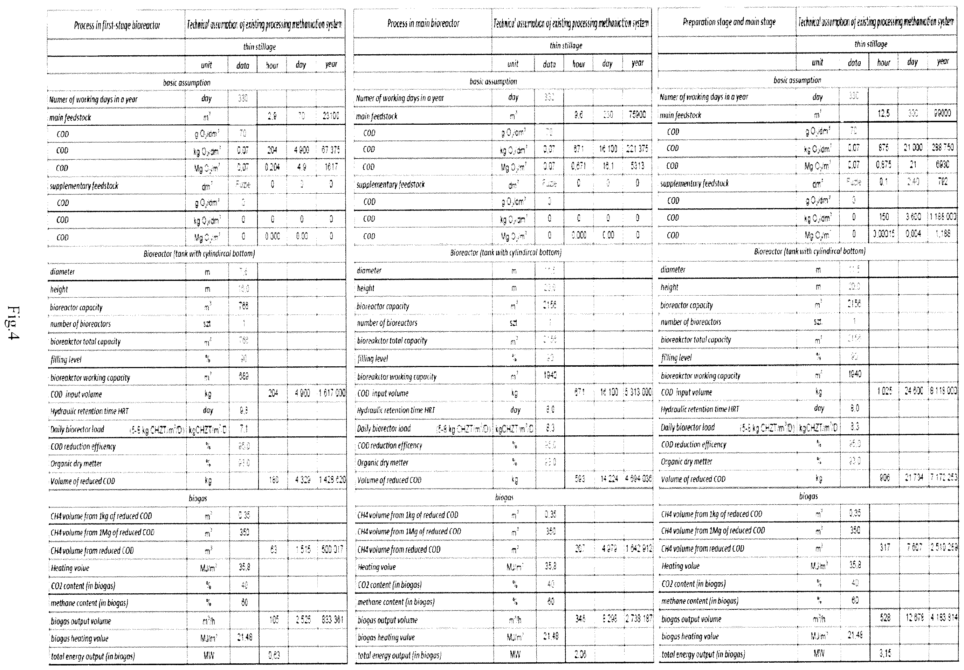

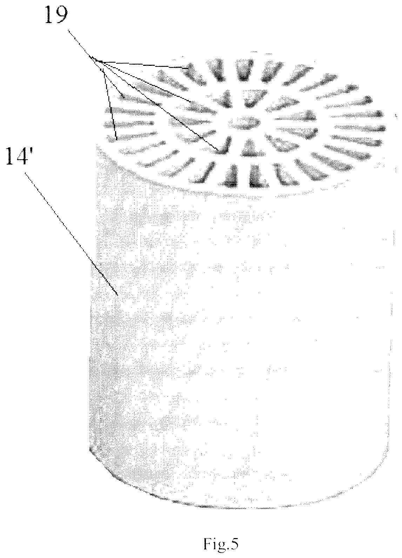

The inventions are presented in the embodiment in the drawing, wherein Fig.1 shows schematically the structure of the reactor for biogas production and wastewater treatment, as well as describes and presents the circulation of medium and the chemical and physical conditions existing and taking place for the method implemented by the structure; Fig. 2 shows a table with values assigned to different key stages of a sample process, by assigning them to measuring points A to J; Fig. 3 shows a table that refers to the points A to J presented in the drawing, which table concerns the course of a sample process in time, considering a full cycle of dosed leachate sample, that is from introducing the sample to producing biogas; and Fig.4 shows comparative tables (the first one for the cascade reactor, the second one for the buffer tank, and the third one for the main tank) which comparatively present the biogas output and the COD reduction, showing the unexpected increment of biogas and unexpected reduction of COD for the cascade arrangement relative to separated tanks and relative to aggregated outputs from separated tanks; and Fig.5 shows schematically a single membrane filter used in the process, including its channels.

A sample method of producing biogas in an anaerobic biological wastewater treatment plant involves the use of hydrolysis, acidogenesis, acetogenesis and methanogenesis. The process is continuous, though carried out in two stages, and uses a substrate supplied upon mixing and temperature stabilisation after the previous basic production stage. The substrate is a centrifugated stillage, that is stillage leachate. At the first stage, the pH is maintained below 7, and at the second stage it is maintained above 7, and at the same time both stages are carried out at 30°C to 45°C, in tanks 3, the first of which is the buffer tank 3' and the second is the main tank 3", and the substrate is gradually moved from the first to the second tank, while continuously making up for its loss in the first tank. The pH is maintained with basic substances or buffers, in order to use such an environment for fermentation among bacteria, so as a result we obtain biogas, methane and carbon dioxide in a proportion of 60/40, being a product of the reaction, which emits an excess biomass. The flow of agents through the tanks 3 and transmission pipelines 2 is controlled by a control system 9, based on the readouts of sensors 4, 5, 6. The first stage, taking place in the buffer tank 3', includes the processes of hydrolysis, acidogenesis and acetogenesis of the leachate, and the leachate temperature during the reaction is stabilised at 38°C +/- 3°C, even though the leachate is supplied to the first tank at 30°C to 35°C (but its temperature after the basic distillation process is as high as 70°C to 80°C), and then, having equalised the flow velocity and concentration and having stabilised the temperature, we force the flow of pre-reacted substrate (ferment) to the second tank (main tank 3"), where it is intensively linked with the retentate in a semi-liquid phase, that is biomass filtered in the system of membrane filters 14 connected to the second tank (main tank 3") by an external forced circuit. The digestate produced in the membrane filter system 14 is introduced to the core 15 running centrally through the second tank, at half height of the core 15, and then the excess concentrated sludge, as a digestate produced from the retentate, is removed from the reactor 1 at the bottom of the core 15, and the digestate of semi-liquid phase is removed by overflow at the upper part of the core 15, thus feeding the second (main) tank 3", and the filtrate, as a treated leachate, is removed from the membrane filter system 14. The leachate introduced to the first (buffer) tank 3' remains therein and reacts for 25h, during which for the first hour it is introduced to the buffer tank 3', and in the twenty-fifth hour it is transmitted to the main tank 3", and then the pre-reacted substrate (ferment), supplied to the second (main) tank 3", is mixed therein and filtered for the next 50h, and then only mixed for the next 50h, during which for the last hour it is removed from the main tank 3" in the concentrated form, and from membrane filters 14 as sewage and filtrate, and at the same time the produced biogas is continuously collected, which is then removed from the tanks 3 in the 124th hour of the process. A full cycle of a dosed leachate sample, from its introduction to the production of biogas, takes 124h. At the first stage, we obtain a pH ranging from 5.5 to 6.5, and at the second stage - from 7 to 7.6, thanks to supplying nutrients (N, P, FeC13) at proportions generally accepted for such a process, and both at the first and second stages we adjust the pH by appropriately supplying NaOH. The filtrate is utilized or returned to the distillery, that is to the previous basic production stage. Biogas is collected every 124h, as already mentioned. The membrane filter system 14 is chemically cleaned once a month, while the process is continued, and the cleaning is carried out using a secondary circuit separated from the system of tanks 3' and 3", with the forced direction being opposite (when running through the membrane filters 14') to the direction of flow through the membrane filters 14' during a standard process.

A sample reactor 1 for biogas production and wastewater treatment includes two tanks 3 interconnected with transmission pipelines 2, the first of which is the buffer tank 3' and the second is the main tank 3", where both tanks are provided with a temperature sensor 4, pH sensor 5 and pressure sensor 6. The tanks 3 are equipped with mixing modules 7 that include a rotor 8. The biogas outlet 23 is located at the top of each tank 3' and 3". The flow of agents through the tanks 3 and transmission pipelines 2 is controlled by a control system 9, based on the readouts of sensors 4, 5, 6. The mixing modules 7 are located outside the tanks 3 and are each time connected to the tanks 3 with their own pipeline circuit system 10, which has a turbulent circulation pump 11, and the main tank 3" is additionally connected to a membrane filter system 14 through an external turbulent pipeline circuit 13 forced by the second pump 12. The membrane filter system 14 is connected by its outlet to the core 15 running centrally through the main tank, at half height of the core 15, which core 15 is opened on top to obtain overflow into the main tank 3", and at the bottom it is terminated with an outlet 16 for concentrated sludge. The membrane filter system 13 has its own outlet 17 for filtrate in the form of treated leachate. The membrane filter system 14 is connected to the main tank 3" by shut-off valves 18. The membrane filter system 14 has a second secondary circuit 13', used for chemical cleaning of the filters. The membrane filter system 14 is provided with three filters 14' in the form of membranes, located in parallel to one another, in vertical position. The cross-section of the membrane filter system 14 has an area of 0.45m². A single membrane filter 14' is longitudinally divided inside into channels 19 having a shape similar to a triangle in the first ring of channels 19, and to a trapezoid in the second ring of channels 19. One filter 14' has a total of thirty-nine channels 19. The capacity of the main tank 3" is no less than twofold and no more than fivefold higher than the capacity of the buffer tank 3', but does not exceed 5,000 m³, in this case the buffer tank 3' has a capacity of 500m³, and the main tank 3" has a capacity of 2000m³. Each of the tanks 3 is provided with an anti-foaming sprinkler 20. Each of the tanks 3 is provided with a sight glass 21, which is equipped with a movable wiper 22. The main tank 3" has two independent membrane filter systems 14, which operate alternately. The membrane filter 14' is a ceramic filter made of titanium oxide, with a throughput of the membrane mesh of no less than 0.1 µm.

## Claims

1. A method of producing biogas in an anaerobic biological wastewater treatment plant, with the use of hydrolysis, acidogenesis, acetogenesis and methanogenesis, which is carried out continuously, though in two stages, using a substrate supplied upon mixing and temperature stabilisation after a previous basic production stage, wherein the substrate is a centrifugated stillage, that is stillage leachate, wherein at the first stage we obtain a pH below 7, and at the second stage we obtain a pH above 7, and both stages are carried out at 30°C to 45°C, in tanks, the first of which is the buffer tank, and the substrate is gradually moved from the first to the second tank, while continuously making up for its loss in the first tank, and the pH is obtained using basic substances or buffers, in order to use such an environment for fermentation among bacteria, so as a result we obtain methane and carbon dioxide, that is biogases being products of the reaction, which emits an excess biomass, **characterised in that** the first stage, taking place in the buffer tank (3'), includes the processes of hydrolysis, acidogenesis and acetogenesis of the leachate, and the leachate temperature during the reaction is stabilised at 38°C +/- 3°C, even though the leachate is supplied to the first (buffer) tank (3') at 30°C to 35°C, and then, having equalised the flow velocity and concentration and having stabilised the temperature, we force the flow of pre-reacted substrate (ferment) to the second (main) tank (3"), where it is intensively linked with the retentate in a semi-liquid phase, that is biomass filtered in the system of membrane filters (14) connected to the second (main) tank (3") by an external forced circuit, wherein the digestate produced in the membrane filter system is introduced to the core (15) running centrally through the second tank (3"), generally at half height of the core (15), and then the excess concentrated sludge, as a digestate produced from the retentate, is removed from the reactor (1) at the bottom of the core (15), and the digestate of semi-liquid phase is removed by overflow at the upper part of the core (15), thus feeding the second (main) tank (3"), and the filtrate, as a treated leachate, is removed from the membrane filter system (14), wherein the leachate introduced to the buffer tank (3') remains therein and reacts for 24h +/- 2h on average, and then the pre-reacted substrate (ferment), supplied to the main tank (3"), is mixed therein and filtered for the next 48h +/- 2h on average, and then only mixed for the next 48h +/- 2h on average, and at the same time the produced biogas is continuously collected, and a full cycle of a dosed leachate sample, from its introduction to the production of biogas, takes less than 125h, where at the first stage we obtain a pH ranging from 5.5 to 6.5, and at the second stage we obtain a pH ranging from 7 to 7.6.

2. The method of biogas production according to claim 1, **characterised in that** at the first or second stage (preferably at the first one), nutrients are supplied, preferably N or P or FeCl3.

3. The method of biogas production according to claim 1, **characterised in that** at the first or second stage the pH is adjusted, preferably by appropriately supplying NaOH.

4. The method of biogas production according to claim 1, **characterised in that** the filtrate is utilized or returned to the distillery, that is to the previous basic production stage.

5. The method of biogas production according to claim 1, **characterised in that** biogas is collected on a one-off basis or biogas is collected in cycles or biogas is collected in portions.

6. The method of biogas production according to claim 1, **characterised in that** the membrane filter system (14) is chemically cleaned at least once a month, while the process is continued, and the cleaning is carried out using a secondary circuit (13') separated from the system of tanks (3' and 3"), with the forced direction being opposite (when running through the membrane filters (14')) to the direction of flow through the membrane filters (14') during a standard process.

7. A reactor for biogas production and wastewater treatment, which has two tanks interconnected with transmission pipelines, the first tank being the buffer tank and the second tank being the main tank, wherein both tanks have a temperature sensor, pH sensor and pressure sensor, and are provided with mixing modules with a rotor, and the biogas outlet is located at the top of each tank, wherein the flow of agents through the tanks and transmission pipelines is controlled by a control system, based on the readouts of sensors, **characterised in that** the mixing modules (7) are located outside the tanks (3) and are each time connected to the tanks (3) with their own pipeline circuit system (10), which has a turbulent circulation pump (11), and the main tank (3") is additionally connected to a membrane filter system (14) through an external turbulent pipeline circuit (13) forced by the second pump (12), wherein the membrane filter system (14) is connected by its outlet to the core (15) running centrally through the main tank (3"), generally at half height of the core (15), which core (15) is opened on top to obtain overflow into the main tank (3"), and at the bottom it is terminated with an outlet (16) for concentrated sludge, and the membrane filter system (14) has its own outlet (17) for filtrate in the form of treated leachate.

8. The reactor according to claim 7, **characterised in that** the membrane filter system (14) is connected to the main tank (3") by shut-off valves (18).

9. The reactor according to claim 7 or 8, **characterised in that** the membrane filter system (14) has a second secondary circuit (13'), used for chemical cleaning of the filters (14').

10. The reactor according to claim 7, **characterised in that** the membrane filter system (14) is provided with at least three filters (14') in the form of membranes, located in parallel to one another, preferably in vertical position.

11. The reactor according to claim 7 or 10, **characterised in that** the cross-section of the membrane filter system (14) has an area of up to 1m².

12. The reactor according to claim 7, **characterised in that** a single membrane filter (14') is longitudinally divided inside into channels (19) having a shape similar to a triangle or trapezoid.

13. The reactor according to claim 12, **characterised in that** a single filter (14') has no less than five and no more than 40 channels (19).

14. The reactor according to claim 7, **characterised in that** the capacity of the main tank (3") is no less than twofold and no more than fivefold higher than the capacity of the buffer tank (3') but does not exceed 5,000 m³.

15. The reactor according to claim 7, **characterised in that** any of the tanks (3', 3") is provided with an anti-foaming sprinkler (20).

16. The reactor according to claim 7, **characterised in that** any of the tanks (3', 3") is provided with a sight glass (21), which is preferably equipped with a movable wiper (22).

17. The reactor according to claim 7, **characterised in that** the main tank (3") has at least two independent membrane filter systems (14).

18. The reactor according to claim 7, **characterised in that** a membrane filter (14') is a ceramic filter, preferably made of titanium or zirconium oxide, with a throughput of the membrane mesh of 0.01 µm to 2.0µm.
